Europäisches Patentamt

European Patent Office

Office européen des brevets

(11) Publication number: **0 385 375**
**A1**

# EUROPEAN PATENT APPLICATION

(21) Application number: 90103752.3

(51) Int. Cl.5 **C07D 211/22**

(22) Date of filing: 26.02.90

(30) Priority: 28.02.89 US 316902

(43) Date of publication of application:
05.09.90 Bulletin 90/36

(84) Designated Contracting States:
AT BE CH DE DK ES FR GB GR IT LI LU NL SE

(71) Applicant: MERRELL DOW
PHARMACEUTICALS INC.
P.O. Box 156300 2110 East Galbraith Road
Cincinnati Ohio 45215-6300(US)

(72) Inventor: McCarty, Frederick J.
7141 Juniperview Lane
Cincinnati, Ohio 45243(US)

(74) Representative: Vossius & Partner
Siebertstrasse 4 P.O. Box 86 07 67
D-8000 München 86(DE)

(54) Preparation of the low melting polymorphic form of terfenadine.

(57) A process for the preparation of the low-melting polymorphic form of terfenadine involving controlled heating and subsequent cooling of a solution of terfenadine in an appropriate solvent.

EP 0 385 375 A1

# PREPARATION OF THE LOW MELTING POLYMORPHIC FORM OF TERFENADINE

Terfenadine, 1-(p-tert-butylphenyl)-4-[4'-(alpha-hydroxydiphenylmethyl)-1'-piperidinyl]butanol, is a non-sedating antihistamine. Previous crystallization procedures for producing terfenadine result in a product which, although analytically pure, nevertheless has a widely variable melting point. Recently, it was discovered that solid terfenadine exists in two distinct crystalline or polymorphic forms, each form having a different melting point. It was further discovered that terfenadine crystallized by the prior art processes varied widely in polymorphic composition and that this polymorphic variation accounted for the observed variation of the melting point of terfenadine.

In an attempt to attain a material with a controlled set of physical properties, which facilitates quality control functions associated with large scale production of terfenadine, a process has recently been developed wherein both polymorphic forms of terfenadine can be produced. (See, U.S. Patent No. 4,742,175, issued May 3, 1988.) This patent concerns mainly the production of the high-melting polymorphic form (Form I) of terfenadine where the terfenadine is digested at reflux in a solvent containing water. However, it additionally claims a process for producing the low-melting polymorphic form of terfenadine (Form II).
According to this process, form II can be attained by dissolving terfenadine in toluene or acetone at about 0° C to about 35° C and allowing the mixture to slowly evaporate over a two-day period.

Applicants have now discovered a new process for preparing the low-melting polymorphic form of terfenadine (Form II) which allows collection of the product in considerably less time than the prior method.

This invention relates specifically to the process which comprises:

a) forming a solution wherein terfenadine is dissolved in a solvent which has been heated to a temperature of from about 40° C to about 80° C, and which is selected from the group consisting of ethyl acetate, chloroform, C₁-C₆ lower alkanol and acetone;

b) cooling the mixture to a temperature of from about 0° C to about 20° C; and

c) collecting the crystalline product. This process is useful for producing the low-melting polymorphic form of terfenadine. This process facilitates quality control functions associated with large scale production of terfenadine, and typically accomplishes the production of Form II with fewer steps and in less time than other methods.

The term "$C_1$-$C_6$ lower alkanol" means any lower alkanol containing one to six carbon atoms in which terfenadine will dissolve. Suitable lower alkanols include methanol, ethanol, n-propanol, isopropanol, isobutanol, amyl alcohol, t-butanol and cyclohexanol. Preferably the lower alkanol solvent used in the preparation of the lower melting polymorph of terfenadine will be methanol, ethanol or isopropanol. Methanol is the most preferred solvent. Mixtures of 2 or more lower alkanols is also contemplated.

Generally, the terfenadine solution is prepared conventionally by simply dissolving terfenadine, which has been prepared by standard methods known in the art, in a solvent that has been heated by standard means to a temperature of from about 40° C to about 80° C. Generally, the elevated temperature is the boiling point of the solvent used, and is maintained at least until terfenadine has been dissolved. Where ethyl acetate is the solvent, the elevated temperature will preferably be about 75° C to about 77° C. Where acetone is the solvent, the elevated temperature will preferably be about 54° C to about 56° C, where a $C_1$-$C_6$ lower alkanol is the solvent, the elevated temperature will preferably be about the boiling point of the solvent/terfenadine solution. For example, where methanol is the solvent, the elevated temperature will preferably be about 64° C. Where chloroform is the solvent, the elevated temperature will preferably be about 60° C to about 62° C.

Preferably, the terfenadine dissolved will be substantially free of impurities. It is also preferable to filter the hot solution prior to crystal formation in order to remove any insoluble, particulate material.

Typically, the solutions of terfenadine in solvent will be highly concentrated, near the saturation point of solute in solvent, although the solution need not be saturated or substantially near saturation for purposes of this invention. Where ethyl acetate is the solvent, for example, the weight to volume ratio of terfenadine to ethyl acetate can be from 80 mg/ml to 300 mg/ml, preferably from, 150 mg/ml to 200 mg/ml. The weight to volume ratio of terfenadine to acetone can be from 30 mg/ml to 250 mg/ml, preferably from, 90 mg/ml to 150 mg/ml. The weight to volume ratio of terfenadine to methanol, for example, can be from 50 mg/ml to 200 mg/ml, preferably from 80 mg/ml to 130 mg/ml. The weight to volume ratio of terfenadine to chloroform can be from 200 mg/ml to 400 mg/ml, preferably from 300 mg/ml to 350 mg/ml. Weight to volume ratios outside of the illustrative ranges above are contemplated as being within the scope of this invention.

Once terfenadine has been dissolved in hot solvent and, if desired, filtered, the solution can be allowed to return to ambient temperature (i.e., about 25°C) by simply removing the heat source. Then the solution can be actively cooled to 0°C to 20°C by standard procedures well-known in the art. Alternatively, once terfenadine has been dissolved in hot solvent and, if desired, filtered, the solution can be immediately actively cooled to a temperature of about 0°C to about 20°C by standard procedures well-known in the art. Such standard cooling procedures include the use of refrigeration and a simple ice-water bath. If desired, methanol or acetone can be added to the cooling bath to lower the temperature even further.

Preferably, when ethyl acetate is the solvent, the solution will be cooled to a temperature of about 5°C to about 15°C, most preferably to about 10°C, before collecting the crystalline product. Preferably, when acetone is the solvent, the solution will be cooled to a temperature of about 0°C to about 10°C, most preferably to about 5°C, before collecting the crystalline product. Preferably, when methanol, for example, is the solvent, the solution will be cooled to a temperature of about 0°C to about 10°C, most preferably to about 5°C before collecting the crystalline product. Preferably, when chloroform is the solvent, the solution will be cooled to a temperature of about 15°C to about 20°C, most preferably to about 20°C, before collecting the crystalline product.

When chloroform is used as the solvent, it is also preferable to cool the terfenadine/chloroform solution to about 40°C and then add about 2 volumes of ether to the solution before cooling.

Methods known in the art may be used with the process of this invention to enhance any aspect of this process. For example, the terfenadine solution may be seeded with one or more crystals of the desired polymorphic product prior to the initiation of product recrystallization.

Recrystallization of the product sufficient for collection will typically be accomplished within about 2 to about 20 hours after cooling. However the length of time required will vary depending on such factors as total volume of solution being processed, size of container, and degree of saturation of the terfenadine solution being processed, method of cooling the solution utilized, amount of product sought to be recovered, and presence or absence of stirring or other well-known methods that can be employed to enhance the recrystallization process. It is contemplated that recrystallization by utilization of this process will be accomplished within time periods outside the illustrative range noted above. The solution can be cooled for any desired period of time. For instance, cooling may continue as a matter of convenience, or it may be specifically for the period of time considered optimal for the desired purpose.

Generally, after cooling the solution, the product can be collected by any standard method known in the art such as by filtration, filtration under vacuum, or decantation and drying. Typically, this product will be collected by filtration when any of the solvents within the scope of this invention are used.

The process of this invention is directed to the production of Form II of terfenadine which is the low-melting polymorphic form of terfenadine characterized to melt at a temperature of about 144°C to about 147.5°C. This form is to be distinguished from Form I, which is the high-melting form and which melts at a temperature of about 149.5°C to about 151°C.

The product recovered by the process of this invention can range from an enriched product to a product which is 100% pure. An enriched product is one that contains at least more than 50% of Form II of terfenadine. Generally, the product recovered by use of the process of this invention will be predominantly Form II, however, the degree of purity will depend on the conditions undertaken to perform the process.

The following specific examples are presented to illustrate the process which is this invention, but they should not be construed as limiting the scope of this invention in any way.

## EXAMPLE 1

## PREPARATION OF TERFENADINE

A one-liter, three-neck flask equipped with a mechanical stirrer, a pressure-equalizing addition funnel and a reflux condenser fitted with nitrogen bubbler was charged with 72.8 g (0.149 mol) of terfenadone monohydrate and 320 ml absolute ethanol. The mixture was heated to reflux, a clear solution formed and a solution of 12.0 g (0.317 mol) of sodium borohydride in 83.2 g of 50% sodium hydroxide and 25.6 ml of water was added dropwise, with stirring, over a period of 1 hour. After the addition was complete, the reaction mixture was stirred at reflux for 2 hours. Terfenadine began to crystallize. The slurry was then diluted with 320 ml of water by adding the water at such a rate that the reaction remained at reflux. The slurry was then cooled slowly to room temperature. The solid was filtered from the slurry, washed with 100 ml of 50/50 (vol/vol) ethanol/water, washed with two 250 ml portions of deionized water, air dried, and vacuum dried at 60°C to give 68.2 g (97% yield) of terfenadine as a mixture of polymorphs.

## EXAMPLE 2

### PREPARATION OF FORM II FROM ETHYL ACETATE SOLUTION

A one liter Erlenmeyer flask, equipped with a magnetic stirrer and a reflux condenser, was charged with 62.3 g of terfenadine from Example 1 and 350 ml ethyl acetate, and the mixture was heated to 75°C while stirring. After the terfenadine was completely dissolved, the resultant solution was filtered through a sintered glass funnel to clarify, and then allowed to cool to ambient temperature, during which time the product began to crystallize. The slurry was then cooled to 10°C by refrigeration and held at that temperature for 19 hours. The solid was then filtered from the slurry and dried at 63°C in vacuo, to yield 56.8 g (91%) low melting polymorph of terfenadine, m.p. 146°C-147°C.

Anal. Calc'd. for $C_{32}H_{41}NO_2$: C: 81.48, H: 8.76, N: 2.97;
Found: C: 81.53, H: 8.76, N: 2.74.

## EXAMPLE 3

### PREPARATION OF FORM II FROM ACETONE SOLUTION

In a manner similar to that of Example 2, 3.0 g of terfenadine was dissolved in 27 ml of acetone heated to 56°C, filtered to clarify and the filtrate cooled to 5°C and held for 17 hours. The crystallized solid was collected by filtration to yield 2.55 g (85%) low melting polymorph of terfenadine, m.p. 145°C-147°C.

## EXAMPLE 4

### PREPARATION OF FORM II FROM METHANOL SOLUTION

In a manner similar to that of Example 3, 410 g or terfenadine containing about 5% water was dissolved in 4 liters of methanol heated to 64°C with stirring in a 5 liter round bottom flask. The solution was filtered to clarify and the filtrate cooled to 5°C for 20 hours. The crystallized solid was collected by filtration to yield 387.2 g (99%) of low melting polymorph of terfenadine, mp 144°C-145°C.

## EXAMPLE 5

### PREPARATION OF FORM II FROM CHLOROFORM SOLUTION

In a similar manner to that of Example 2, 386.5 g of terfenadine was dissolved in 1.2 liters of chloroform heated to 61°C with stirring in a 2 liter round bottom flask. The hot solution was treated with 35 g of charcoal and allowed to cool while stirring for 3 hours. The mixture was then again heated to 61°C and filtered through diatomaceous earth. The filtrate was allowed to cool to ambient temperature and diluted with 2.8 liters of ether, whereupon the product began to crystallize. The slurry was cooled to a temperature of 18°C and held at that temperature for 16 hours. The solid product was then collected by filtration, and allowed to air dry to yield 259.3 g (67%) of low melting polymorph, m.p. 145°C-146°C.

It should be apparent to one of ordinary skill in the art that changes and modifications can be made to this invention without departing from the spirit or scope of the invention as it is set forth herein.

## Claims

1. A process for producing a low-melting polymorphic form of terfenadine (Form II) which comprises:
forming a solution wherein terfenadine is dissolved in a solvent which has been heated to a temperature of from 40°C to 80°C, and which is selected from the group consisting of ethyl acetate, chloroform, $C_1$-$C_6$ lower alkanol, and acetone, and then crystallizing the product.

2. A process according to Claim 1 wherein, after dissolving terfenadine in said solvent, the solution is cooled to a temperature of from 0°C to 20°C.

3. A process according to any of Claims 1 or 2 wherein the solvent is ethyl acetate.

4. A process according to any of Claims 1 to 3 wherein the solvent is heated to a temperature of from 75°C to 77°C.

5. A process according to any of Claims 1 to 4 wherein, when the solution is formed, the weight to volume ratio of terfenadine to solvent is 80 mg/ml to 300 mg/ml.

6. A process according to any of Claims 1 or 3 to 5 wherein the solution is cooled to a temperature of 5°C to 15°C.

7. A process according to any of Claims 1 or 2 wherein the solvent is acetone.

8. A process according to Claim 7 wherein the solvent is heated to a temperature of from 54°C to 56°C.

9. A process according to Claim 7 wherein, when the solution is formed, the weight to volume ratio of terfenadine to solvent is 30 mg/ml to 250 mg/ml.

10. A process according to Claim 7 wherein the solution is cooled to a temperature of 0°C to 10°C.

11. A process according to any of Claims 1 or 2 wherein the solvent is $C_1$-$C_6$ lower alkanol.

12. A process according to Claim 11 wherein the lower alkanol solvent is methanol.

13. A process according to Claim 12 wherein the weight to volume ratio of terfenadine to methanol is 50 mg/ml to 200 mg/ml, and the solution is heated to a temperature of from 62°C to 65°C, then cooled to a temperature of from 0°C to 10°C.

14. A process according to any of Claims 1 or 2 wherein the solvent is chloroform.

15. A process for producing a low-melting polymorphic form of terfenadine which comprises:

a) forming a solution wherein terfenadine is dissolved in chloroform which has been heated to a temperature of from 40°C to 62°C;

b) adding ether to said solution;

c) crystallizing the product.

| DOCUMENTS CONSIDERED TO BE RELEVANT | | | EP 90103752.3 |
|---|---|---|---|
| Category | Citation of document with indication, where appropriate, of relevant passages | Relevant to claim | CLASSIFICATION OF THE APPLICATION (Int. Cl.⁵) |
| D,A | US - A - 4 742 175 (FAWCETT) * Claims 9, 10; example 1 * -- | 1,6,7 | C 07 D 211/22 |
| A | GB - A - 1 413 138 (RICHARDSON-MERRELL) * Example 1 * ---- | 1,7 | |
| | | | TECHNICAL FIELDS SEARCHED (Int. Cl.⁵) |
| | | | C 07 D 211/00 |

The present search report has been drawn up for all claims

| Place of search | Date of completion of the search | Examiner |
|---|---|---|
| VIENNA | 10-05-1990 | HOCHHAUSER |

EPO Form 1603 03.82